(19) Europäisches Patentamt
European Patent Office
Office européen des brevets

(11) Publication number: **0 028 277**
**B1**

(12) # EUROPEAN PATENT SPECIFICATION

(45) Date of publication of patent specification: **23.01.85**

(51) Int. Cl.⁴: **C 07 C 79/46,** A 01 N 37/48, C 07 C 121/75

(21) Application number: **79302436.5**

(22) Date of filing: **02.11.79**

(54) **Substituted diphenyl ethers, herbicidal compositions containing them and method of combating weeds.**

(43) Date of publication of application:
**13.05.81 Bulletin 81/19**

(45) Publication of the grant of the patent:
**23.01.85 Bulletin 85/04**

(84) Designated Contracting States:
**BE CH DE FR GB IT NL**

(56) References cited:
**DE-A-2 525 399**
**DE-A-2 753 900**
**US-A-3 928 416**

(73) Proprietor: **Rohm and Haas Company**
**Independence Mall West**
**Philadelphia, Pennsylvania 19105 (US)**

(72) Inventor: **Johnson, Wayne Orrin**
**346 Centennial Road**
**Warminster Pennsylvania 18974 (US)**
Inventor: **Swithenbank, Colin**
**1710 West Rock Road**
**Perkasie Pennsylvania 18944 (US)**
Inventor: **Yih, Roy Yang Ming**
**94 Windover Lane**
**Doylestown Pennsylvania 18901 (US)**

(74) Representative: **Wood, Peter Worsley Spencer et al**
**Rohm and Haas Company Patent Department**
**Chesterfield House Bloomsbury Way**
**London WC1A 2TP (GB)**

**EP 0 028 277 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention concerns novel substituted diphenyl ethers which show activity as herbicides, herbicidal compositions which contain these compounds, and methods of controlling, i.e. combating weeds.

Certain substituted diphenyl ethers have been shown to be effective weed control agents, such as those of U.S. Patent No. 3,928,416 which discloses herbicidal substituted diphenyl ethers of the formula:

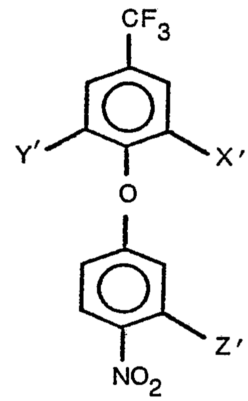

wherein

X′ is hydrogen, halogen, trihalomethyl, alkyl or cyano;
Y′ is hydrogen, halogen or trihalomethyl; and
Z′ is, *inter alia,* alkoxycarbonyl.

The herbicidal effectiveness of a given diphenyl ether cannot be predicted from an examination of the substituent groups attached to the phenyl rings in the ether, and often quite closely related compounds will have quite different weed control abilities. Various diphenyl ethers may have overlapping or complementary areas of activity or selectivity, and can thus be useful in combination to control a variety of weeds upon application of a single composition. Furthermore, the diphenyl ethers heretofore disclosed as herbicides are not completely effective. An ideal herbicide should give selective weed control, over the full growing season, with a single administration at low rates of application. It should be able to control all common weeds by killing them as the seed, the germinating seed, the seedling, and the growing plant. At the same time, the herbicide should not be phytotoxic to the crops to which it is applied and should decompose or otherwise be dissipated so as not to poison the soil permanently. The known diphenyl ether herbicides fall short of these ideals, and it would thus be desirable to have new herbicides which show even more selective control of undesirable plants among desirable crop plants or which complement known herbicides in activity.

In accordance with the present invention, there is provided a class of novel substituted diphenyl ethers having the formula:

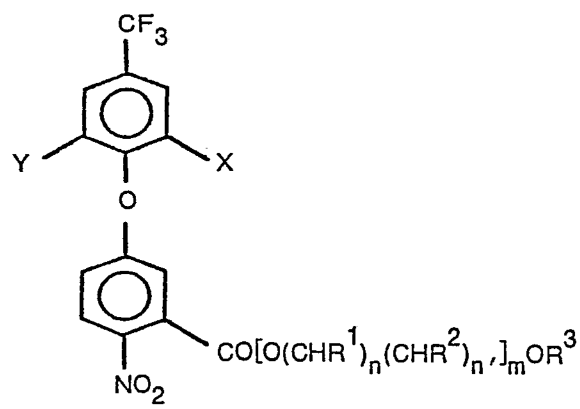

(I)

wherein

X is hydrogen, halogen, preferably fluorine or chlorine, trifluoromethyl, $(C_1—C_4)$alkyl (preferably methyl), or cyano;
Y is hydrogen or halogen (preferably fluorine or chlorine);
$R^1$ is hydrogen or $(C_1—C_4)$ alkyl (preferably methyl);
$R^2$ is hydrogen or $(C_1—C_4)$alkyl (preferably methyl);

$R^3$ is hydrogen or $(C_1—C_4)$alkyl (preferably methyl);

m is an integer of 1 to 50 or, in the case of a mixture, an average number greater than 1 and not more than 50;

n is 0 or an integer of 1 to 6; and

n' is 0 or an integer of 1 to 6, provided that the sum of n and n' is an integer of 2 to 6 (preferably 2).

When the X, $R^1$, $R^2$, or $R^3$ substituent is an alkyl group, it can have either a straight- or branched-chain configuration.

These novel compounds have been found to show unexpected activity particularly as regards selectivity as weed control agents. In a preferred embodiment of the invention, X is a halogen atom, most preferably a chlorine atom, Y is a hydrogen atom or a halogen atom, preferably a chlorine atom, and either $R^1$ or $R^2$ is a hydrogen atom. Most preferably both $R^1$ and $R^2$ are hydrogen atoms.

Examples of the compounds of the invention embraced by Formula I include:

2-Chloro-4-trifluoromethyl-3'-(2-methoxy)ethoxycarbonyl-4'-nitrodiphenyl ether
2-Chloro-4-trifluoromethyl-3'-(2-hydroxy)ethoxycarbonyl-4'-nitrodiphenyl ether
4-Trifluoromethyl-3'-(ω-methoxypentaethoxy)carbonyl-4'-nitrodiphenyl ether
2-Bromo-4-trifluoromethyl-3'-(ω-hydroxypentaethoxy)carbonyl-4'-nitrodiphenyl ether
2,4-Bis-trifluoromethyl-3'-(ω-hydroxydecaethoxy)carbonyl-4'-nitrodiphenyl ether
2,6-Dichloro-4-trifluoromethyl-3'-(ω-hydroxydecaethoxy)carbonyl-4'-nitrodiphenyl ether
2-Fluoro-6-chloro-4-trifluoromethyl-3'-(ω-propoxypentadecaethoxy)carbonyl-4'-nitrodiphenyl ether
2-Methyl-4-trifluoromethyl-3'-(ω-hydroxypentadecaethoxy)carbonyl-4'-nitrodiphenyl ether
2-Cyano-4-trifluoromethyl-3'-(ω-isobutoxypentadecaethoxy)carbonyl-4'-nitrodiphenyl ether
2-Iodo-4-trifluoromethyl-3'-(ω-*t*-butoxyeicosaethoxy)carbonyl-4'-nitrodiphenyl ether
4-Trifluoromethyl-3'-(ω-hydroxyeicosaethoxy)carbonyl-4'-nitrodiphenyl ether
2-Chloro-4-trifluoromethyl-3'-(ω-methoxypentaethoxypenta(α-methyl)ethoxy)carbonyl-4'-nitrophenyl ether
2-Chloro-4-trifluoromethyl-3'-(ω-hydroxyhexamethylene)oxycarbonyl-4'-nitrodiphenyl ether
4-Trifluoromethyl-3'-(2-methoxypropoxy)carbonyl-4'-nitrodiphenyl ether
2-Fluoro-6-bromo-4-trifluoromethyl-3'-(3-ethyl-4-ethoxybutoxy)carbonyl-4'-nitrodiphenyl ether

The novel substituted diphenyl ethers of the invention are useful both as preemergence and as postemergence herbicides. Preemergence herbicides are ordinarily used to treat the soil in which the desired crop is to be planted by application either before seeding, during seeding, or, as in most applications, after seeding and before the crop emerges. Post-emergence herbicides are those which are applied after the plants have emerged and during their growth period. Compounds of the invention are particularly active against broadleaf weeds.

Among the crops on which the diphenyl ethers of the invention can be advantageously employed are, for example, soybeans, corn (i.e. maize), wheat, barley, rice and other cereal crops.

When used in transplanted rice crops, the ethers can be applied either preemergence or postemergence to the weeds—that is, they can be applied to the growth medium of the transplanted plants either before the weed plants have emerged or while they are in their early stages of growth. The ethers can be applied to the growth medium either before or after the rice has been transplanted to that medium.

The diphenyl ethers of the invention can be applied in any amount which will give the desired degree of weed control. A preferred rate of application of the diphenyl ethers of the invention is from about 0.112 to about 13.45 kg/ha and most preferably about 0.28 to about 4.48 kg/ha (i.e. about 0.1 to 12, preferably 0.25 to 4 lbs/acre).

Under some conditions, the diphenyl ethers of the invention may be advantageously incorporated into the soil or other growth medium prior to planting a crop. This incorporation can be carried out by any convenient means, including by simple mixing with the soil, by applying the diphenyl ether to the surface of the soil and then discing or dragging into the soil to the desired depth, or by employing a liquid carrier to accomplish the necessary penetration and impregnation.

A diphenyl ether of the invention can be applied to the growth medium or to plants to be treated either by itself or, as is generally done, as a component in a herbicidal composition or formulation which also comprises an agronomically acceptable carrier. By agronomically acceptable carrier is meant any substance which can be used to dissolve, disperse or diffuse a herbidical compound in the composition without impairing the effectiveness of the herbicidal compound and which by itself has no detrimental effect on the soil, equipment, crops, or agronomic environment. Mixtures of the diphenyl ethers of the invention may be used in any of these herbicidal formulations. The herbicidal compositions of the invention can be either solid or liquid formulations or solutions. For example, the diphenyl ethers can be formulated as wettable powders, water-dispersible pastes, emulsifiable concentrates, dusts, granular formulations, aerosols, or flowable emulsion concentrates. In such formulations, the compounds are extended with a liquid or solid carrier and, when desired, suitable surfactants are incorporated. Guidance as to formulations and the mode of application of the diphenyl

ethers may be derived from U.S. Patent No. 3,928,416 referred to above. The diphenyl ethers of the invention can be used in conjunction with other herbicides, or fertilizers, such as those listed in U.S. Patent No. 3,928,416.

The following tabulation gives the usual and preferred concentrations in percentages by weight of the active ingredient (i.e. one or more of the diphenyl ethers of the invention) in various formulations.

| Formulation | Usual active ingredient concentration | Preferred active ingredient concentration |
|---|---|---|
| Wettable powder | 1—90 | 20—50 |
| Emulsifiable concentrate | 1—90 | 20—50 |
| Flowable emulsion concentrate | 1—60 | 40—50 |
| Ready-to-use dust | 0.1—50 | 1—10 |
| Dust concentrate | 10—90 | 40—50 |
| Granules | 0.1—30 | 1—10 |
| Pastes | 1—80 | 20—50 |

The ethers of the invention can be prepared by esterification or transesterification of a diphenyl ether of the formula:

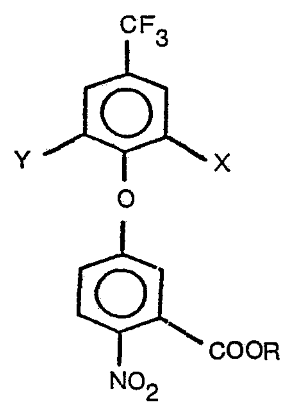

(II)

wherein X and Y are as defined above, and R is a hydrogen atom or an alkyl group, or of the corresponding acid chloride, with an alcohol of the formula:

$$H—[O(CHR^1)_n(CHR^2)_{n'}]_mOR^3 \qquad (III)$$

wherein $R^1$, $R^2$, $R^3$, m, n and n′ are as defined above. One method which is commonly used for preparing the carbinols of Formula III is the polymerization or copolymerization of the corresponding alkylene oxides by conventional techniques. The esterification is generally carried out at a temperature of about 0 to about 200°C, and preferably at about 100 to about 150°C, optionally in the presence of an inert organic solvent, such as a hydrocarbon or chlorinated hydrocarbon. In the esterification reaction, one equivalent of the alcohol is generally employed, although excess alcohol can be used as a solvent in the reaction. When the acid chloride is used as the reagent, an acid scavenger may be used. Typical acid scavengers include inorganic bases, such as potassium hydroxide, and amines, such as 2,6-lutidine.

The free acid can also be esterified using a trialkylorthoformate, without any additional acid catalyst. Other conventional esterification techniques, such as transesterification in the presence of an acid catalyst, can also be used.

A modification of the above preparative method for preparing the esters of the invention involves the reaction of a metal salt, such as the sodium salt, or an acid of Formula II with a halide of the formula:

$$Hal[(CHR^1)_n(CHR^2)_{n'}O]_mR^3 \qquad (IV)$$

wherein $R^1$, $R^2$, $R^3$, m, n and n′ are as defined above and Hal is a halogen, preferably chlorine or bromine, atom. This reaction is generally carried out at a temperature of about 0 to about 200°C, preferably about 100 to about 150°C, and in an inert organic solvent, such as benzene or toluene.

The diphenyl ether precursors can be prepared by reacting a suitably substituted phenol, or the potassium or sodium salt of the phenol, with a suitably substituted halobenzene, such as a chloro- or fluoro-benzene in the presence of an alkaline agent. Such precursors and their preparation are described in U.S. Patent No. 3,928,416 already referred to.

Another route to the compounds of the invention involves the nitration of a diphenyl ether of the formula:

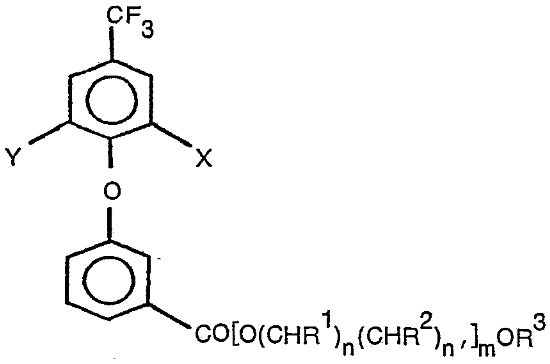

(V)

wherein X, Y, and $R^1$, $R^2$, $R^3$, m, n and n′ are as defined above, using typical nitrating agents such as potassium nitrate in sulfuric acid, acetyl nitrate, mixed sulfuric acid/nitric acid and nitrosonium tetrafluoroborate. The nitration reaction is generally carried out at about —20 to about 100°C, preferably about 0 to about 5°C, optionally in the presence of an inert organic solvent, such as methylene chloride or other chlorinated hydrocarbon.

Diphenyl ethers of the invention can also be prepared by condensing a phenol of the formula:

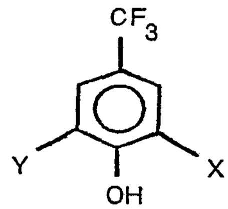

(VI)

wherein X and Y are as defined above with a substituted halobenzene of the formula:

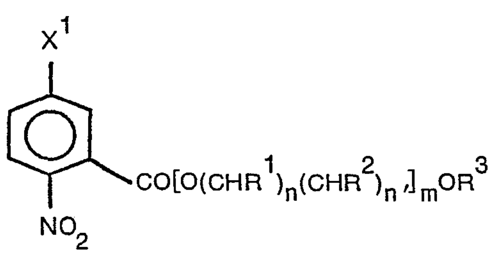

(VII)

wherein $X^1$ is a halogen atom, preferably a fluorine atom, and $R^1$, $R^2$, $R^3$, m, n and n′ are as defined above. This reaction is generally carried out at a temperature of about 0 to about 250°C, preferably about 75 to about 200°C, optionally in the presence of an appropriate solvent, such as sulfolane, dimethylsulfoxide, dimethylformamide, hexamethylphosphorustriamide, or other inert polar organic solvent.

The following Examples will further illustrate the preparation of compounds according to this invention. In Table I following the Examples, typical diphenyl ethers of the invention are listed together with their elemental analyses. In Table I, "- -" indicates "not determined".

Example 1

Preparation of 2-chloro-4-trifluoromethyl-3′-(2-methoxy)ethoxycarbonyl-4′-nitrodiphenyl ether (Compound No. 2, Table I)

a) Preparation of 2-methoxyethyl-*m*-fluorobenzoate

A solution *m*-fluorobenzoyl chloride (15.8 g 0.1 mole) and 2-methoxyethanol (8.4 g 0.11 mole) is heated in glyme (100 ml) for 8 hours at 60°C. The solvent is removed under reduced pressure and the residue distilled to give 9.3 g of 2-methoxyethyl-*m*-fluorobenzoate b.p. 70—75°C/26,7 $Nm^{-2}$ (0.2 mmHg).

b) Preparation of 2-methoxyethyl-5-fluoro-2-nitrobenzoate

2-Methoxyethyl-*m*-fluorobenzoate (9.3 g 0.047 mole) is added dropwise at 5°C to concentrated sulfuric acid (100 ml). A solution of potassium nitrate (4.7 g 0.047 mole) in concentrated sulfuric acid (25 ml) is then added, the mixture is stirred at room temperature for two hours, and then poured on to ice. Extraction with carbon tetrachloride followed by drying and stripping of the solution yields 2-methoxyethyl-5-fluoro-2-nitrobenzoate, which on recrystallization from ligroin/benzene has a melting point of 49.5—50.5°C.

c) Preparation of 2-chloro-4-trifluoromethyl-3′-(2-methoxy)ethoxycarbonyl-4′-nitrodiphenyl ether

A mixture of 2-chloro-4-trifluoromethylphenol (3.92 g, 0.02 mole) and potassium carbonate (2.89 g, 0.02 mole) is stirred overnight in dimethyl sulfoxide (50 ml) at room temperature. 2-Methoxyethyl-5-fluoro-2-nitrobenzoate (4.86 g, 0.02 mole) is added and the mixture stirred overnight, poured onto ice, and extracted with carbon tetrachloride. The extract is stripped to give 2-chloro-4-trifluoromethyl-3′-(2-methoxy)ethoxycarbonyl-4′-nitrodiphenyl ether (7.5 g) which solidifies on standing, and has a melting point of 60—62°C.

Example 2

Preparation of 2-chloro-4-trifluoromethyl-3′-(ω-methoxyhexadecaethoxy)carbonyl-4′-nitrodiphenyl ether (Compound No. 4, Table I)

A solution of 2-chloro-4-trifluoromethyl-3′-chlorocarbonyl-4′-nitrodiphenyl ether (9.5 g, 0.025 mole) in toluene (300 ml) is heated under reflux for 4 hours in the presence of polyethylene glycol monomethyl ether, of average molecular weight 750 (19 g, 0.025 mole). The toluene is then removed under reduced pressure to give 26 g of 2-chloro-4-trifluoromethyl-3′-(ω-methoxy-hexadecaethoxy)-carbonyl-4′-nitrodiphenyl ether.

Example 3

Preparation of 2-chloro-4-trifluoromethyl-3′-(ω-methoxydotetracontaethoxy)carbonyl-4′-nitrodiphenyl ether (Compound No. 5, Table I)

A solution of 2-chloro-4-trifluoromethyl-3′-chloro-carbonyl-4′-nitrodiphenyl ether (0.48 g, 0.0013 mole) in toluene (300 ml) is heated under reflux for 4 hours in the presence of polyethylene glycol monomethyl ether, of average molecular weight 1900 (2.5 g, 0.0013 mole). The toluene is then removed under reduced pressure to give 2.4 g of 2-chloro-4-trifluoromethyl-3′-(ω-methoxy dotetracontaethoxy)carbonyl-4′-nitrodiphenyl ether.

In Table I that follows,

*indicates liquid which decomposed on attempted distillation

**indicates average number.

TABLE I

Diphenyl ethers—physical data

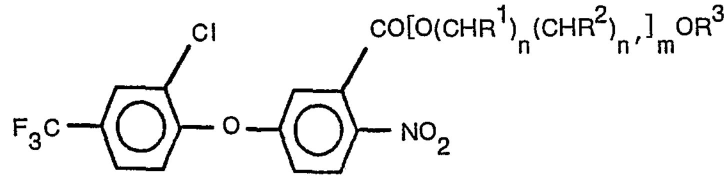

| Compound No. | $R^1$ | n | $R^2$ | n' | m | $R^3$ | m.p. | Found (calculated) %C | %H | %N | %Cl | %F |
|---|---|---|---|---|---|---|---|---|---|---|---|---|
| 1 | H | 2 | — | 0 | 1 | H | 68—70°C | 47.18 (47.37) | 2.72 (2.73) | 3.29 (3.45) | 8.58 (8.74) | 16.99 (14.05) |
| 2 | H | 2 | — | 0 | 1 | $CH_3$ | 60—62°C | 48.40 (48.64) | 3.23 (3.12) | 3.17 (3.33) | 8.68 (8.44) | 13.18 (13.57) |
| 3 | H | 2 | — | 0 | 2 | $C_2H_5$ | * | 48.61 (50.29) | 3.61 (4.01) | 3.19 (2.93) | 8.73 (7.42 | 12.29 (11.93) |
| 4 | H | 16** | — | 0 | 1 | $CH_3$ | * | 51.93 (53.10) | 7.21 (6.90) | 1.40 (1.30) | -- (--) | -- (--) |
| 5 | H | 42** | — | 0 | 1 | $CH_3$ | * | 52.99 (54.30) | 8.67 (7.21) | 0.87 (0.64) | -- (--) | -- (--) |
| 6 | H | 2 | — | 0 | 10 | H | * | -- (--) | -- (--) | -- (--) | -- (--) | -- (--) |
| 7 | $CH_3$ | 1 | H | 1 | 1 | $CH_3$ | * | 50.00 (49.85) | 3.47 (3.46) | 3.83 (3.23) | 8.24 (8.17) | 13.27 (13.14) |
| 8 | H | 2 | — | 0 | 7 | $CH_3$ | * | 50.60 (50.91) | 5.92 (5.42) | 2.16 (2.05) | 5.78 (5.18) | 7.54 (8.33) |
| 9 | H | 2 | — | 0 | 12 | $CH_3$ | * | 51.26 (51.80) | 6.56 (6.35) | 1.38 (1.55) | 4.59 (3.92) | 5.76 (6.30) |
| 10 | H | 4 | — | 0 | 1 | $CH_3$ | * | 50.67 (50.96) | 3.82 (3.83) | 3.33 (3.13) | 8.08 (7.92) | 12.39 (12.73) |

The following examples show the herbicidal properties of diphenyl ethers of the invention.

Example 4

This example shows the herbicidal activity of two diphenyl ethers (Compound Nos. 1 and 2, Table I) towards a number of common weeds. Using the procedure described below, the diphenyl ethers were evaluated for control of various plant species.

The following test procedure was employed. Seeds of selected crops and weeds were planted in soil in flats. For preemergence tests, the flats were treated with the test compound immediately after the planting. For postemergence tests, the seeds were allowed to germinate, and after two weeks the flats were treated with the test compound immediately after the planting. The compound to be evaluated was dissolved in acetone, diluted with water, and sprayed over the flats using a carrier volume equivalent to 468 l/ha (i.e. 50 U.S. gallons per acre) at a rate of application of 2.24 kg/ha (i.e. two pounds per acre). About two weeks after the application of the test compound, the state of growth of the plants was observed and the phytotoxic effect of the compound was evaluated. The results of typical tests are summarized in Table II, which gives the average percent control achieved by the test compounds with 0 representing no weed control and 100 complete kill of the plant.

TABLE II
Herbicidal activity

| Plant | Compound No. 2 | | Compound No. 1 | |
|---|---|---|---|---|
| | Pre-emergence | Post emergence | Pre-emergence | Post emergence |
| *Echinochloa crusgalli* | 100 | 100 | 99 | 100 |
| *Bromus tectorum* | 100 | 90 | 90 | 40 |
| *Setaria viridis* | 100 | 100 | 100 | 100 |
| *Sorghum halepense* | 100 | 100 | | |
| *Cyperus esculentus* | 50 | 100 | 90 | 70 |
| *Agropyron repens* | 0 | 40 | 0 | 40 |
| *Avena fatua* | 70 | 99 | 90 | 99 |
| *Cynodon dactylon* | | 60 | | 0 |
| *Panicum miliaceaum* | | | 100 | 100 |
| *Convolvulus arvensis* | 0 | 100 | 50 | 100 |
| *Xanthium pensylvanicum* | 0 | 100 | 50 | 0 |
| *Sesbania* spp. | 100 | 100 | 30 | 100 |
| *Ipomoea* spp. | 0 | 100 | 0 | 100 |
| *Solanum nigrum* | 100 | 100 | 100 | 100 |
| *Abutilon theophrasti* | 40 | 100 | 100 | 100 |
| *Carthamus tinctorius* | 0 | 99 | 30 | 100 |

Example 5

Compounds 1 to 3 of Table I were also evaluated in conventional field tests. In one typical field test. Compound 2 was tested for control of a number of annual grasses and broadleaf weeds and for tolerance to a number of common crops. Table III summarizes these tests results, using the same scale of control as in Table II.

TABLE III
Field test activity

| Common name | Scientific name | TEI* Rate (kg/ha) | Pre-emergence 71 0.28 | 1.12 | Early post emergence 28 0.13 | 0.56 | Post emergence 26 0.13 | 0.56 |
|---|---|---|---|---|---|---|---|---|
| lambsquarters | *Chenopodium album* | | 10 | 92 | 77 | 98 | 22 | 99 |
| velvetleaf | *Abutilon theophrasti* | | 75 | 100 | 98 | 100 | 100 | 100 |
| fall panicum | *Panicum dichotomiflorum* | | 0 | 32 | 30 | 60 | 0 | 7 |
| pigweed | *Amaranthus retroflexus* | | 50 | 100 | 100 | 100 | 100 | 100 |
| soybean | *Glycine max* | | 0 | 2 | 0 | 32 | 5 | 25 |
| rape | *Brassica napus* | | 10 | 99 | 0 | 93 | 10 | 85 |
| rice | *Oryza sativa* | | 5 | 5 | 12 | 10 | 12 | 20 |
| cotton | *Gossypium hirsutum* | | 10 | 99 | 95 | 100 | 22 | 90 |
| peanut | *Arachis hypogaea* | | 5 | 15 | 49 | 0 | 5 | 12 |
| sugarbeet | *Beta vulgaris* | | 42 | 100 | 85 | 100 | 35 | 95 |
| sunflower | *Helianthus annuus* | | 35 | 65 | 22 | 75 | 25 | 35 |
| wheat | *Triticum* spp. | | 12 | 12 | 15 | 27 | 20 | 25 |
| barley | *Hordium* spp. | | 0 | 0 | 2 | 7 | 2 | 0 |
| corn | *Zea mays* | | 0 | 0 | 0 | 7 | 12 | 12 |
| tomato | *Lycopersicum esculentum* | | 7 | 97 | | | | |

*Days between treatment and evaluation.

Compounds 6—10 (Table I) were evaluated as herbicides in the manner described in Example 4 except that two rates of application of the compounds were employed. The beneficial results obtained are given in Table IV.

TABLE IV
Herbicidal activity

| Weed | Kg/ha | Compound No. 6 Pre-emergence | Compound No. 6 Post emergence | Compound No. 7 Pre-emergence | Compound No. 7 Post emergence | Compound No. 8 Pre-emergence | Compound No. 8 Post emergence | Compound No. 9 Pre-emergence | Compound No. 9 Post emergence | Compound No. 10 Pre-emergence | Compound No. 10 Post emergence |
|---|---|---|---|---|---|---|---|---|---|---|---|
| Monocots | | | | | | | | | | | |
| *Echinochloa crusgalli* | 2.24[1] | | | 99 | 70 | 80 | | 70 | 70 | 80 | |
| | 4.48 | | | 100 | 80 | 90 | 90 | 95 | 70 | 100 | |
| *Bromus tectorum* | 2.24 | | | 80 | 99 | | | | | 90 | |
| | 4.48 | | | 80 | 99 | | | | | 100 | 70 |
| *Setaria viridis* | 2.24 | | | 100 | 95 | 100 | 100 | 99 | 90 | 100 | 95 |
| | 4.48 | 100 | 80 | 100 | 95 | 95 | 100 | 100 | 100 | 100 | 80 |
| *Sorghum halepense* | 2.24 | | | 95 | 80 | 99 | 80 | 99 | 90 | 99 | |
| | 4.48 | | | 100 | 90 | 99 | 90 | 100 | 95 | 100 | |
| *Cyperus esculentus* | 2.24 | | | | | | | | 80 | | |
| | 4.48 | | | | | | | | 90 | | |
| *Avena fatua* | 2.24 | | | 80 | | | | | | | |
| | 4.48 | | | 80 | | 70 | 80 | | 70 | 80 | |
| Dicots | | | | | | | | | | | |
| *Xanthium pensylvanicum* | 2.24 | | 100 | | 100 | | 100 | | 100 | 90 | 100 |
| | 4.48 | | 100 | 70 | 100 | | 100 | | 100 | 99 | 99 |
| *Ipomoea* spp. | 2.24 | | 100 | 100 | 100 | 95 | 100 | 100 | 100 | 100 | |
| | 4.48 | | 100 | 100 | 100 | 95 | 100 | 95 | 100 | 100 | 100 |
| *Abutilon theophrasti* | 2.24 | | 95 | 100 | 95 | 80 | 100 | 80 | 100 | 100 | 80 |
| | 4.48 | | 95 | 100 | 95 | 70 | 100 | 90 | 100 | 100 | 95 |

[1]=Equivalent to 2 lbs/acre.

## Claims

1. 4-Trifluoromethyl-3'-substituted-4'-nitrodiphenyl ethers optionally containing one or two additional ring substituents in the 2- or 5-positions, characterised in that the diphenyl ethers are of the formula:

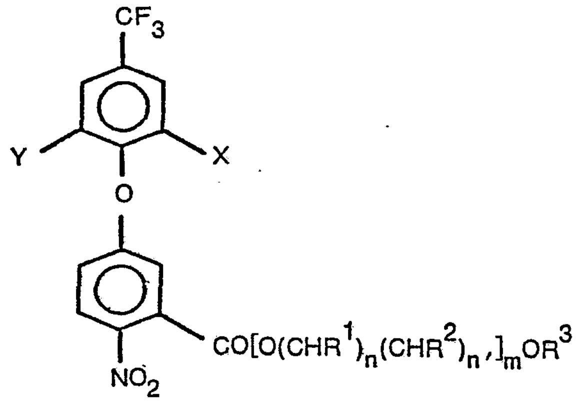

(I)

wherein

X is hydrogen, halogen, $(C_1—C_4)$alkyl, cyano or trifluoromethyl;

Y is hydrogen or halogen;

$R^1$ is hydrogen or $(C_1—C_4)$alkyl;

$R^2$ is hydrogen or $(C_1—C_4)$alkyl;

$R^3$ is hydrogen or $(C_1—C_4)$alkyl;

m is an integer of 1 to 50 or, in the case of a mixture, an average number greater than 1 and not more than 50;

n is 0 or an integer of 1 to 6;

n' is 0 or an integer of 1 to 6, provided that the sum of n and n' is an integer of 2 to 6.

2. Compounds according to claim 1, wherein X is chlorine and Y is hydrogen.

3. Compounds according to claim 2, wherein $R^3$ is a hydrogen atom or a methyl group and the sum of n and n' is 2.

4. A compound according to claim 3, wherein m is 1 or 2.

5. A herbicidal composition which comprises one or more compounds according to any one of claims 1 to 4, an agronomically acceptable carrier and, optionally, a surfactant.

6. A method of combating weeds which comprises applying to weed seedlings, or to the surface of a growth medium prior to the emergence of the weeds from the growth medium, one or more compounds according to any of claims 1 to 4, the compound(s) being applied in an amount effective to combat the growth of the weeds.

7. A method according to claim 6 as applied to the selective combating of weeds in an agronomic crop area.

## Patentansprüche

1. 4-Trifluormethyl-3'-subst.-4'-nitrodiphenylether, die gegebenenfalls einen oder zwei zusätzliche Ringsubstituenten in der 2- oder 5-Position enthalten, dadurch gekennzeichnet, daß die Diphenylether der Formel

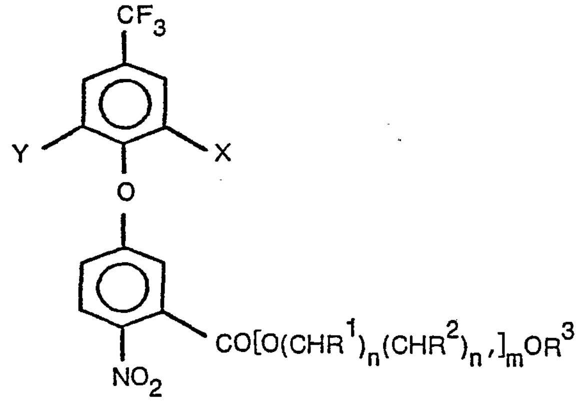

(I)

entsprechen, worin

X für Wasserstoff, Halogen, ($C_1$—$C_4$)Alkyl, Cyano oder Trilfuormethyl steht,

Y Wasserstoff oder Halogen ist,

$R^1$ Wasserstoff oder ($C_1$—$C_4$)Alkyl ist,

$R^2$ Wasserstoff oder ($C_1$—$C_4$)Alkyl bedeutet,

$R^3$ Wasserstoff oder ($C_1$—$C_4$)Alkyl versinnbildlicht,

m eine ganze Zahl von 1 bis 50 oder im Falle einer Mischung mit einer durch schnittlichen Zahl von mehr als 1 nicht mehr als 50 ist,

n 0 oder eine ganze Zahl von 1 bis 6 ist,

n' 0 oder eine ganze Zahl von 1 bis 6 ist, unter der Voraussetzung, daß die Summe von n und n' eine ganze Zahl von 2 bis 6 ist.

2. Verbindungen nach Anspruch 1, dadurch gekennzeichnet, daß X Chlor ist und Y Wasserstoff bedeutet.

3. Verbindungen nach Anspruch 2, dadurch gekennzeichnet, daß $R^3$ ein Wasserstoffatom oder eine Methylgruppe ist und die Summe von n und n' 2 ist.

4. Verbindung nach Anspruch 3, dadurch gekennzeichnet, daß m für 1 oder 2 steht.

5. Herbizides Mittel aus einer oder mehreren Verbindungen gemäß einem der Ansprüche 1 bis 4 und einem für landwirtschaftliche Zwecke verträglichen Träger und gegebenenfalls einem grenzflächenaktiven Mittel.

6. Verfahren zum Bekämpfen von Unkräutern, dadurch gekennzeichnet, daß auf die Unkrautsämlinge oder auf die Oberfläche eines Wachstumsmediums vor dem Auflaufen der Unkräuter aus dem Wachstumsmedium eine oder mehrere Verbindung(en) gemäß einem der Ansprüche 1 bis 4 aufgebracht werden, wobei die Verbindung(en) in einer Menge aufgebracht wird (werden), die zur Bekämpfung des Wachstums der Unkräuter wirksam ist.

7. Verfahren nach Anspruch 6, dadurch gekennzeichnet, daß es auf die selektive Bekämpfung von Unkräutern an einer Stelle angewendet wird, auf der Nutzpflanzen wachsen.

**Revendications**

1. Ethers de 4-trifluorométhyl-3' substitués-4'-nitro-diphényle, les cas échéant contenant un ou deux substituants cycliques supplémentaires en position 2 ou 5, caractérisés en ce que les éthers de diphényle répondent à la formule

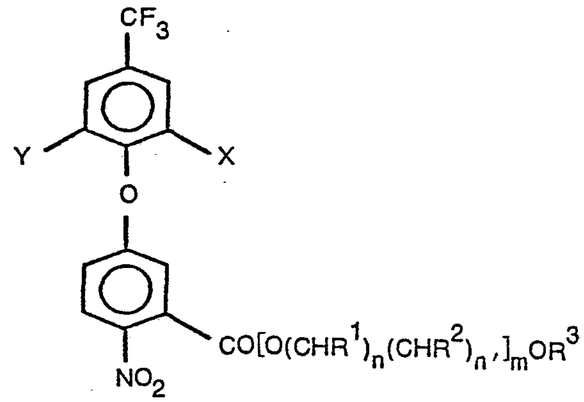

(I)

dans laquelle:

X est un hydrogène, un halogène, un alcoyle en $C_1$ à $C_4$, un cyano ou un trifluorométhyle;

Y est un hydrogéne ou un halogène;

$R^1$ est un hydrogène ou un alcoyle en $C_1$ à $C_4$;

$R^2$ est un hydrogène ou un alcoyle en $C_1$ à $C_4$;

$R^3$ est un hydrogène ou un alcoyle en $C_1$ à $C_4$;

m est un entier de 1 à 50 ou, dans le cas d'un mélange, correspond à une moyenne et est supérieur à 1 et ne dépasse pas 50;

n est 0 ou un entier de 1 à 6;

n' est 0 ou un entier de 1 à 6, sous réserve que la somme de n et n' soit un entier de 2 à 6.

2. Composés selon la revendication 1, caractérisés en ce que X est du chlore et Y de l'hydrogène.

3. Composés selon la revendication 2, caractérisés en ce que $R^3$ est un atome d'hydrogène ou un groupe méthyle et que la somme de n et de n' est 2.

4. Composé selon la revendication 3, caractérisé en ce que m est 1 ou 2.

5. Composition herbicide caractérisée en ce qu'elle comprend un ou plusieurs composés selon l'une quelconque des revendications 1 à 4, un véhicule agronomiquement acceptable et, le cas échéant, un agent tensio-actif.

6. Procédé pour lutter contre les mauvaises herbes, caractérisé en ce qu'il comprend l'application aux pousses de mauvaises herbes ou à la surface d'un milieu de croissance avant l'apparition des mauvaises herbes du milieu de croissance, d'un ou de plusieurs composés selon l'une quelconque des revendications 1 à 4, le(s) composé(s) étant appliqué(s) en une quantité efficace pour lutter contre la croissance des mauvaises herbes.

7. Procédé selon la revendication 6, tel qu'appliqué pour lutter sélectivement contre les mauvises herbes dans une zone de culture agronomique.